(19) **Europäisches Patentamt / European Patent Office / Office européen des brevets**

(11) **EP 4 549 593 A2**

(12) # EUROPEAN PATENT APPLICATION

(43) Date of publication:
**07.05.2025 Bulletin 2025/19**

(21) Application number: **25163692.4**

(22) Date of filing: **28.07.2022**

(51) International Patent Classification (IPC):
**C12R 1/125** [(2006.01)]

(52) Cooperative Patent Classification (CPC):
**C12N 1/20; C05C 1/00; C05C 5/00; C05F 11/08; C12N 1/205; C12N 9/0044; C12Y 107/01014; C12Y 107/02002;** C12R 2001/11; C12R 2001/125; Y02P 60/21

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Validation States:
**MA TN**

(30) Priority: **28.07.2021 EP 21382702**

(62) Document number(s) of the earlier application(s) in accordance with Art. 76 EPC:
**22758448.9 / 4 377 441**

(71) Applicant: **Fertiberia, S.A.**
**28036 Madrid (ES)**

(72) Inventors:
• **GONZALEZ ANDRES, Fernando**
  **24004 León (ES)**
• **BARQUERO QUIROS, Marcia Paulina**
  **24004 León (ES)**
• **CARPINTERO SALVO, José Manuel**
  **28036 Madrid (ES)**
• **BRAÑAS LASALA, Javier**
  **28036 Madrid (ES)**
• **LAUREANO MARÍN, Ana Maria**
  **28036 Madrid (ES)**

(74) Representative: **Hoffmann Eitle**
**Hoffmann Eitle S.L.U.**
**Paseo de la Castellana 140, 3a planta**
**Edificio LIMA**
**28046 Madrid (ES)**

Remarks:
This application was filed on 14.03.2025 as a divisional application to the application mentioned under INID code 62.

(54) **BIOLOGICAL COMPOSITION FOR NITRATE LEACHING PREVENTION**

(57) The present invention relates to microorganism compositions and fertilisers comprising them, which reduce soil nitrate accumulation and their use for avoiding nitrogen leaching.

**EP 4 549 593 A2**

## Description

This application is a divisional application of the EP application 22 758 448.9.

### FIELD OF THE INVENTION

The present invention belongs to the field of agriculture. The present invention relates to microorganism compositions and fertilisers comprising them, which reduce soil nitrate accumulation and their use for avoiding nitrogen leaching.

### BACKGROUND OF THE INVENTION

Leaching is the entrainment of nitrate with the flow or drainage of water through the soil profile. As a consequence, nitrogen is transported to deeper layers, being out of reach of the roots of the crops. Nitrate is soluble in water and does not interact with soil colloids, being very mobile, and this is the reason why nitrate suffer the leaching process, reaching groundwater and surface water, causing the problem of water pollution. By contrast, ammonium ions are adsorbed by the soil's colloids, preventing from leaching.

The nitrogen provided by a fertiliser has multiple destinations when it is incorporated into the soil. The rates of use of nitrogen by the crop rarely exceed 60 or 70 %. In addition, there is a need for nitrogen fertilisers of greater efficiency, for example, slow release, encouraged by tendencies of the environment in relation to nitrogen losses and agronomic reasons in relation to the need for nitrogen throughout the crop cycle. This objective can be achieved by using inhibitors of urease and nitrification. Inhibitors of urease and nitrification are the supplements most used in mineral nitrogen fertilisers intended to improve their agronomic performance and to reduce the emission and leachate of contaminant substances. Some of the main inhibitors of nitrification identified so far are dicyanamide, 3-methylpyrazole, 1-guanyl-3-methylpyrazole nitrate, 3,4-dimethylpyrazole, 3,4-dimethylpyrazole phosphate, 1 H-1 ,2,4-triazole, 3-amino-1 H-1 ,2,4-triazole, 4-chloro-3-methylpyrazole and nitrapyrin. Some of the main inhibitors of urease activity identified so far are phenyl phosphorodiamidate, N-(n-Butyl) Phosphoric Diamide, N-(n-Butyl) Thiophosphoric T riamide, N-diaminophosphoryl)-N'-(4-methoxyphenyl)-urea, N-diaminophosphoryl-urea, N-diaminophosphoryl)-N'-phenyl-urea, methyl diaminophosphorylcarbamate, benzyl diaminophosphorylcarbamate, isopropyl diaminophosphorylcarbamate and ammonium thiocyanate.

In agricultural systems, there are a series of interactions and processes that transform and transport nitrogen, all of which are included in the so-called nitrogen cycle. Nitrogen is found in different forms within this cycle. The largest source and reserve of nitrogen is the $N_2$ gas that constitutes 78 % of the Earth's atmosphere. However, higher plants absorb nitrogen in the form of $NO_3^-$ and $NH_4^+$ from the soil solution primarily. The incorporation of gaseous nitrogen into the soil occurs through the fixation processes, where the gaseous nitrogen is incorporated through microorganisms, symbiotic and non-symbiotic, present in the soil (biological fixation), dry and wet deposition of nitrogen compounds (in storms and with atmospheric dust), industrial processes for the synthesis of nitrogen fertilisers (industrial fixation). In the fixation process, $N_2$ is reduced, in most cases, to $NH_3$. Incorporated nitrogen accumulates in the soil mainly organically, assuming more than 90 % of the nitrogen present in the soil. Organic forms are not directly assimilated by plants, but they become so after undergoing their transformation into mineral nitrogen. The main inorganic forms are ammonium ($NH_4^+$), nitrite ($NO_2^-$) and nitrate ($NO_3^-$), normally representing between 2 and 5% of the total nitrogen in the soil. The transformation of organic nitrogen to mineral nitrogen occurs through the mineralization that takes place in several stages, firstly, the breakdown of large protein molecules (aminization) and then, through ammonification, it is transformed into $NH_4^+$. Ammonium can be used directly by plants, nitrified, immobilized, volatilized or retained in the soil exchange complex.

Nitrification is the oxidation of ammonium to nitrate and is basically carried out in two stages through bacterial action. In the first, bacteria oxidize ammonia to nitrite ($NO_2^-$). In the second, nitrite is oxidized to nitrate. In contrast to $NH_4^+$ which is retained by the soil change complex, $NO_2^-$ and $NO_3^-$ are mobile in the soil solution. Thus, these ions are either absorbed together with the soil water by the roots of the plants, or they are gradually leached with the drainage water through the soil profile.

$NO_3^-$ can be reduced by microbes in the soil. There are different metabolic routes for nitrates reduction, namely, denitrification, dissimilatory nitrate reduction to ammonium (DNRA) and assimilatory nitrate reduction to ammonium (ANRA).

Also, there are losses of nitrogen from the soil due to the conversion of inorganic forms to gases that pass into the atmosphere. These nitrogen outputs are by volatilization and denitrification. Volatilization is the process by which ammonia nitrogen is lost to the atmosphere as $NH_3$ (volatile gas) from aqueous solutions. Denitrification is the transformation of nitrate and nitrite in the soil into gases such as $N_2O$ or $N_2$ by bacteria and under anaerobic conditions. Nitrous oxides (NO,O) are incomplete products of the transformation of nitrate to $N_2$ and, as greenhouse gases, contribute to climate change.

There is a need for fertilisers which prevent nitrogen leaching and are environmentally friendly.

## BRIEF DESCRIPTION OF THE DRAWINGS

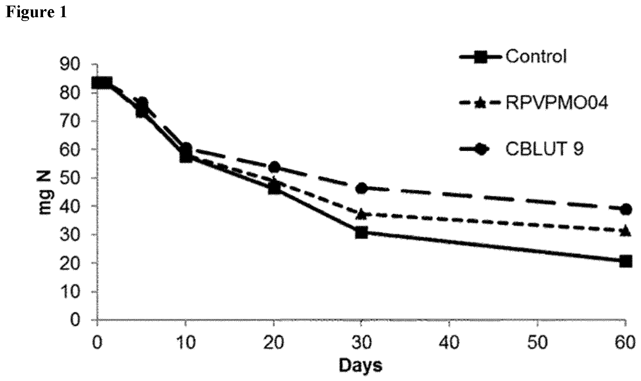

**Figure 1.** Evolution of the total mineral nitrogen in the soil treated with each one of strains CBLUT9 *(Bacillus subtilis)* and RPVPMO04 *(Bacillus megaterium)* or untreated (control), over 60 days.

## SUMMARY

The present invention provides a solution to the problem of nitrogen leaching in crop soils. Moreover, the provided solution is environmentally friendly. The inventors have found that aerobic rhizospheric microorganisms capable of reducing nitrates to ammonium are efficient in reducing nitrogen leaching from soil when added to the soil, alone or in combination with a fertiliser.

Thus, in a first aspect, the present invention relates to composition comprising at least one aerobic rhizospheric microorganism, wherein said microorganism comprises in its genome at least one nitrate reductase gene and at least one nitrite reductase gene and wherein said microorganism reduces nitrate to ammonium when cultured in a minimum growth medium with nitrate as only source of nitrogen.

In a second aspect, the present invention relates to a fertiliser comprising a composition according to the first aspect.

In a third aspect, the present invention relates to the use of the composition of the first aspect or of the fertiliser of the second aspect, for preventing nitrogen leaching and/or for improving general crop performance or crop yield.

In a fourth aspect, the present invention relates to the use of the composition of the first aspect or of the fertiliser of the second aspect, for preventing the emission of $N_2O$.

## DESCRIPTION

The present invention provides a natural solution to the problem of nitrogen leaching so the use of chemical nitrification inhibitors in crop soil can be avoided.

In respect of the microorganism of the composition of the first aspect of the present invention, the term "aerobic", refers to a microorganism that can survive and grow in an oxygenated environment, either facultative (it can survive and grow alternatively in an oxygenated or unoxygenated environment) or strict (it only can survive and grow in an oxygenated environment). The term "rhizospheric", as used herein, refers to the microorganism having been isolated from the Rhizosphere. In a preferred embodiment, the microorganism is a Plant Growth Promoting Rhizobacteria (PGPR).

The nitrate reductase gene is a nucleotide sequence codifying for an enzyme capable of transforming nitrate into nitrite. The nitrite reductase gene is a nucleotide sequence codifying for an enzyme capable of transforming nitrite into ammonium. In a preferred embodiment, the nitrate reductase and nitrite reductase genes in the microorganism's genome are expressed. The production of ammonium is tested in a minimum growth medium: 0.6 g/l KH2PO4, 0.4 g/l $MgSO_4$ $7H_2O$, 4 g/l glucose, 8 g/l mannitol, 8 g/l sodium pyruvate, 1 ml/l vitamins solution and 1 ml/l trace elements (Bergersen et al., 1961 Aust J Biol Sci 14: 349-360), plus 1 g/l KNO3 as the only source of nitrogen. The test is carried out in duplicate, as follows: in test tubes with 5 ml of the growth medium, the microorganism is inoculated and incubated at 28 °C for 72 h. The presence of ammonium is tested with a colorimetric assay based on the Nessler Reagent ($K_2HgI$) which in a strongly alkaline solution reacts with ammonia to produce a yellow-coloured complex in direct proportion to the ammonia concentration, such as the colorimetric commercial test VACUettes KIT Ammonia, CHEMetrics.

In a preferred embodiment of the first aspect, the composition comprises from $10^3$ to $5\cdot10^{12}$ CFU per gram of composition, preferably from $10^5$ to $10^{11}$ CFU per gram of composition, more preferably from $10^6$ to $10^{10}$ CFU per gram of composition.

In a preferred embodiment of the first aspect, the microorganism does not comprise in its genome the gene nrfA. This gene is considered a marker of DNRA. Thus, it is preferred that the microorganism performs ANRA instead of DNRA.

In a preferred embodiment of the first aspect, the microorganism cannot produce $N_2$ from nitrates. The production of nitrogen $N_2$ from nitrates of the microorganism is tested as follows: the microorganism is grown in the classical growth medium for nitrate reduction assays, consisting of 5 g/l peptone, 3 g/l meat extract and 1 g/l potassium nitrate as nitrogen source and incubated at 28 °C for 48 hours. A denitrifying microorganism reduces the nitrate ($NO_3^-$) present in the growth medium to gaseous nitrogen and, therefore, a denitrifying microorganism can be detected by the absence of nitrate and nitrite after the incubation. For this purpose, after the incubation, the microorganism suspension is treated with the reactive NIT 1 (0.8 g of sulphanilic acid + 100 ml acetic acid 5N) plus NIT 2 (0,6 g N-N-dimethyl-1-naphtylamine + 100 ml acetic acid 5N) that stains nitrites in red color. If the sample is not stained, then it is treated with zinc dust, that stains nitrates in red color. In consequence, in the case of denitrifying microorganisms the sample is not stained in any of the two staining steps.

In a preferred embodiment of the first aspect, the microorganism comprises at least one of genes nasB or nasC,

and at least one of genes nasD and nasE. In a preferred embodiment, genes nasB, nasC, nasD and nasE are expressed by the microorganism and thus, their expression can be detected by, for example, RT qPCR.

In a preferred embodiment of the first aspect, the microorganism is gram +.

The composition according to any one of the preceding claims, wherein said microorganism is a species of the *Bacillus genus,* preferably said microorganism is of the species *Bacillus subtilis* or *Bacillus megaterium.* In a preferred embodiment, said microorganism is strain CECT 30572 or CECT 30573, or a combination thereof. In another aspect, the present invention relates to the use of the composition of the first aspect, preferably wherein the microorganism is strain CECT 30572 or CECT 30573, or a combination thereof, for preventing nitrogen leaching, for improving general crop performance or crop yield, or for preventing the emission of $N_2O$.

In respect of the fertiliser of the second aspect of the present invention, the term "fertiliser" refers to a natural or artificial substance containing chemical elements that improve growth and productiveness of plants. In a preferred embodiment, the fertiliser is selected from the group consisting of nitrogenated fertilisers, phosphated fertilisers, potassium fertilisers, NP compound fertilisers, PK compound fertilisers, NK compound fertilisers or NPK compound fertilisers, limestone amendments, magnesium amendments, sulphur amendments, calcium and sulphur amendments, moisture retainer amendments, silica amendments, organic amendments and other soil conditioners or soil correctors. In a preferred embodiment, the fertiliser is solid or liquid, mineral, organo-mineral or organic. In a preferred embodiment, the fertiliser is selected from the group consisting of phosphated fertilisers, potassium fertilisers, NP compound fertilisers, PK compound fertilisers, NK compound fertilisers or NPK compound fertilisers. Typically, nitrogenated fertilisers are applied to the soil at 10 to 500 kg of N/ha.

In a preferred embodiment of the fertiliser of the second aspect, the fertiliser comprises from $10^2$ to $10^{10}$ CFU per gram of fertiliser, preferably from $10^3$ to $10^9$ CFU per gram of fertiliser, more preferably from $10^4$ to $10^8$ CFU per gram of fertiliser. In a preferred embodiment, the fertiliser is applied to the soil so that from 10 to $10^4$ CFU/ g of soil are applied.

In a preferred embodiment of the fertiliser of the second aspect, the microorganism in the composition is protected by a microorganism-protective-compound, such as trehalose, carob gum or xanthan gum. The term "microorganism-protective-compound", as used herein, refers to a compound that enables the microorganism survival under the physiochemical conditions of the fertiliser when the microorganism survival is tested according to the Most Probable Number method, as described in EP3085679.

In a preferred embodiment of the third aspect of the present invention, the composition or fertiliser is directly applied to the soil as is or in combination with an organic or inorganic carrier e.g. peat, biochar, clays, or the composition is applied to the soil in the irrigation water or in a compost or in a soil improver, conditioner or amendment, or the composition is applied to a seed before seeding.

The present invention refers to the following items:

1. A fertiliser comprising a composition comprising at least one aerobic rhizospheric microorganism, wherein said microorganism comprises in its genome at least one nitrate reductase gene and at least one nitrite reductase gene and wherein said microorganism reduces nitrate to ammonium when cultured in a minimum growth medium with nitrate as only source of nitrogen, and wherein the fertiliser is selected from the group consisting of nitrogenated fertilisers, phosphated fertilisers, potassium fertilisers, NP compound fertilisers, PK compound fertilisers, NK compound fertilisers or NPK compound fertilisers, limestone amendments, magnesium amendments, sulphur amendments, calcium and sulphur amendments, moisture retainer amendments, silica amendments, and organic amendments.

2. The fertiliser according to the preceding item, wherein the composition comprises from $10^3$ to 5, $10^{12}$ CFU per gram of composition, preferably from $10^5$ to $10^{11}$ CFU per gram of composition, more preferably from $10^6$ to $10^{10}$ CFU per gram of composition.

3. The fertiliser according to any one of the preceding items, wherein said microorganism does not comprise in its genome the gene nrfA.

4. The fertiliser according to any one of the preceding items, wherein said microorganism cannot produce $N_2$ from nitrates.

5. The fertiliser according to any one of the preceding items, wherein said microorganism comprises at least one of genes nasB or nasC and at least one of genes nasD or nasE.

6. The fertiliser according to any one of the preceding items, wherein said microorganism is gram +.

7. The fertiliser according to any one of the preceding items, wherein said microorganism is a species of the *Bacillus genus,* preferably said microorganism is of the species *Bacillus subtilis* or *Bacillus megaterium.*

8. The fertiliser according to any one of the preceding items, wherein said microorganism is strain CECT 30572, strain CECT 30573 or a combination of both.

9. The fertiliser according to any one of the preceding items, wherein the fertiliser comprises from $10^2$ to $10^{10}$ CFU per gram of fertiliser, preferably from $10^3$ to $10^9$ CFU per gram of fertiliser, more preferably from $10^4$ to $10^8$ CFU per gram of fertiliser.

10. The fertiliser according to any one of the preceding items, wherein the fertiliser is selected from the group consisting of phosphated fertilisers, potassium fertilisers, NP compound fertilisers, PK compound fertilisers, NK compound fertilisers or NPK compound fertilisers.

11. The fertiliser according to any one of the preceding items, wherein the fertiliser is solid or liquid, mineral, organo-mineral or organic.

12. The fertiliser according to any one of the preceding items, wherein the microorganism is protected by a microorganism-protective-compound, such as trehalose, carob gum or xanthan gum.

13. Use of a composition comprising at least one aerobic rhizospheric microorganism, wherein said microorganism comprises in its genome at least one nitrate reductase gene and at least one nitrite reductase gene and wherein said microorganism reduces nitrate to ammonium when cultured in a minimum growth medium with nitrate as only source of nitrogen, or of the fertiliser according to any one of items 1 to 12, for preventing nitrogen leaching and/or for improving general crop performance or crop yield.

14. Use of the composition or fertiliser according to the preceding item, wherein said composition or fertiliser is directly applied to the soil as is or in combination with an organic or inorganic carrier, or wherein said composition is applied to the soil in the irrigation water or in a compost or in a soil improver, or wherein said composition is applied to a seed before seeding.

15. Use of a composition comprising at least one aerobic rhizospheric microorganism, wherein said microorganism comprises in its genome at least one nitrate reductase gene and at least one nitrite reductase gene and wherein said microorganism reduces nitrate to ammonium when cultured in a minimum growth medium with nitrate as only source of nitrogen, or of the fertiliser according to any one of items 1 to 12, for preventing the emission of $N_2O$.

16. A composition comprising at least one aerobic rhizospheric microorganism, wherein said microorganism comprises in its genome at least one nitrate reductase gene and at least one nitrite reductase gene and wherein said microorganism reduces nitrate to ammonium when cultured in a minimum growth medium with nitrate as only source of nitrogen, wherein said microorganism is strain CECT 30572, strain CECT 30573 or a combination of both.

17. Use of the composition according to item 16 for preventing nitrogen leaching, for improving general crop performance or crop yield, or for preventing the emission of $N_2O$.

The present invention also refers to the following items:

18. A fertiliser comprising a composition comprising at least one aerobic rhizospheric microorganism, wherein said microorganism comprises in its genome at least one nitrate reductase gene and at least one nitrite reductase gene, wherein said at least one microorganism does not comprise in its genome the gene nrfA, and wherein said microorganism reduces nitrate to ammonium when cultured in a minimum growth medium with nitrate as only source of nitrogen, and wherein the fertiliser is selected from the group consisting of nitrogenated fertilisers, phosphated fertilisers, potassium fertilisers, NP compound fertilisers, PK compound fertilisers, NK compound fertilisers or NPK compound fertilisers, limestone amendments, magnesium amendments, sulphur amendments, calcium and sulphur amendments, moisture retainer amendments, silica amendments, and organic amendments, characterized in that the at least one aerobic rhizospheric microorganism is the microorganism strain CECT 30572, the microorganism strain CECT 30573 or a combination of both microorganism strains.

19. The fertiliser, according to item 1, wherein the composition comprises from $10^3$ to $5.10^{12}$ CFU per gram of composition.

20. The fertiliser, according to any one of the preceding items, wherein said microorganism cannot produce $N_2$ from nitrates; and/or wherein said microorganism comprises at least one of genes nasB or nasC and at least one of genes nasD or nasE; and/or wherein said microorganism is gram +; and/or wherein said microorganism is a species of the *Bacillus genus.*

21. The fertiliser, according to any one of the preceding items, wherein said microorganism is of the species *Bacillus subtilis* or *Bacillus megaterium.*

22. The fertiliser, according to any one of the preceding items, wherein the fertiliser comprises from $10^2$ to $10^{10}$ CFU per gram of fertiliser.

23. The fertiliser according to any one of the preceding items, wherein the fertiliser is selected from the group consisting of nitrogenated fertilisers, phosphated fertilisers, potassium fertilisers, NP compound fertilisers, PK compound fertilisers, NK compound fertilisers or NPK compound fertilisers.

24. The fertiliser according to any one of the preceding items, wherein the fertiliser is solid or liquid, mineral, organo-mineral or organic.

25. A fertiliser comprising at least the microorganism strain CECT 30572, the microorganism strain CECT 30573 or a combination of both microorganism strains.

26. Use of the fertiliser, according to any of the preceding items, for preventing nitrogen leaching and/or for improving general crop performance or crop yield.

27. Use, according to item 9, wherein said fertiliser is directly applied to the soil as is or in combination with an organic or inorganic carrier, or wherein said composition is applied to the soil in the irrigation water or in a compost or in a soil improver, or wherein said composition is applied to a seed before seeding.

28. Use, according to items 9 or 10, of a fertiliser comprising at least one aerobic rhizospheric microorganism, wherein said microorganism comprises in its genome at least one nitrate reductase gene and at least one nitrite reductase gene and wherein said microorganism reduces nitrate to ammonium when cultured in a minimum growth medium with nitrate as only source of nitrogen for preventing the emission of $N_2O$.

29. A composition comprising at least one aerobic rhizospheric microorganism, wherein said microorganism comprises in its genome at least one nitrate reductase gene and at least one nitrite reductase gene and wherein said microorganism reduces nitrate to ammonium when cultured in a minimum growth medium with nitrate as only source of nitrogen, wherein said microorganism is strain CECT 30572 or a combination of strain CECT 30572 and strain CECT 30573.

30. Use of the composition according to items 12 for preventing nitrogen leaching, for improving general crop performance or crop yield, or for preventing the emission of $N_2O$.

**EXAMPLES**

The following examples illustrate the present invention:

**Microorganisms useful for preventing nitrate leaching**

Two bacteria strains (CBLUT9 *(Bacillus subtilis)* and RPVPMO04 *(Bacillus megaterium))* were isolated from the Rhizosphere. These strains were deposited at the Spanish Type Culture Collection (Colección Española de Cultivos Tipo (CECT)) with deposit numbers CECT 30572 and CECT 30573, respectively. Both microorganisms are gram +, aerobic and reduce nitrate to ammonium when grown with nitrate as only source of nitrogen. Also, none of these microorganisms produce $N_2$ from nitrates when tested according to the method described above.

The whole genome of the two microorganisms was sequenced. For that, the bacterial strains were grown on Tryptic Soy Agar (TSA, Merck) plates during 24 h at 28$^e$C. The genomic DNA was obtained using the bacterial genomic DNA isolation kit (NORGEN®),
following the manufacturer's protocol. Sequencing, upon preparation of pair-end libraries, was performed on Illumina MiSeq sequencing platform (2x250bp) by Microbes NG (United Kingdom).

To search for specific genes in the genome of the strains CBLUT9 *(Bacillus subtilis)* and RPVPMO04 *(Bacillus megaterium),* the software Geneious Prime® 2020.2.4 (Biomatters Ltd.) was used.

None of these microorganisms comprise in their genomes the nrfA gene, which is the marker of the DNRA, showing that this metabolic route is not present in any one of these microorganisms, which were subjected to genome mining in order to search for the presence of the gene nrfA.

The sequence of the gene nrfA was obtained from NCBI database from *Bacillus* species and it was searched in the corresponding genomes, with the tool Blast (Megablast) from Geneious Prime. The results are shown in Table 1.

**Table 1.** Absence of gene nrfA; reaction: nitrite > ammonium; metabolic pathway: DNRA; source: Uniprot or KEGG2020.

| Gene name | Protein name | Source of the gene sequence | Similarity of the sequence of the gene *nrfA* with sequences of the indicated strains | |
|---|---|---|---|---|
| | | | CBLUT9 (*Bacillus subtilis*) | RPVPMO04 (*Bacillus megaterium*) |
| *nrfA* | Nitrogen assimilation transcription factor nirA (Uniprot)<br><br>Nitrite reductase (cytochrome c-552) (KEGG) [EC:1.7.2.2] | *Bacillus azotoformans* LMG9581. Accession number: AJLR01000037.1 Gene position: 9926-11395<br><br>*Bacillus bataviensis* LMG 21833, whole genome. Accession number: AJLS01000123.1 Gene position: 39386-40837 *Bacillus selenitireducens* MLS10. Accession number: CP001791.1: Gene position: 1494856-1496307 | <5% (absent) | <5% (absent) |
| | | *Bacillus beveridgei* strain MLTeJB, complete genome. Accession number:CP012502.1 Gene position: 904052-905503<br><br>*Bacillus vireti* LMG 21834. Accession number: ALAN01000129.1 Gene position: 14648-16099 | | |

**Nitrate/nitrite reductase genes**

The bacterial strains CBLUT9 *(Bacillus subtilis)* and RPVPMO04 *(Bacillus megaterium)* were subjected to genome mining in order to search for the presence of nitrate reductase and nitrite reductase genes involved in the metabolic pathway ANRA.

The whole genome sequence was obtained as indicated above and the search for the presence of specific genes in the problem strains was carried out with the same tool and with the same procedure previously described.

**Table 2.** Presence of genes involved in the ANRA pathway; reaction: nitrite to ammonium or nitrate to ammonium. Source KEGG 2020 or GenBank.

| Gene name | Protein name | Source of the gene sequence | Similarity* | |
| --- | --- | --- | --- | --- |
| | | | CBLUT9 (*Bacillus subtilis*) | RPVPMO04 (*Bacillus megaterium*) |
| *NIT-6* | Nitrite reductase [NAD(P)H] [EC:1.7.1.4] | *Bacillus subtilis* subsp. *subtilis* 168 Accession number (KEGG2020): BSU03300 | *nasD* (large subunit) 99,7% (present) | |
| | | *Bacillus subtilis* subsp. subtilis 168: Accession number (KEGG2020): BSU03290 | *nasE* (small subunit) 99.1% (present) | |
| | | *Bacillus megaterium* QM B1551, complete genome Accession number (GenBank) P001983.1 | | *nasD* (large subunit) 98.3% |

| | | Gene position: 1138543-1140957 | | (present) and 99,2% |
|---|---|---|---|---|
| | | *Bacillus megaterium* QM B1551, complete genome Accession number (GenBank): P001983.1 Gene position: :1140976-1141302 | | *nasE* (small subunit) 99,2% (present) |
| *nasB* C | Nitrate reductase | *Bacillus megaterium* QM B1551, complete genome Accession number (GenBank): CP001983.1: Gene position: 740879-743224 | *nasB* (large subunit) 98.5% (present) | |
| | | *Bacillus megaterium* QM B1551, complete genome Accession number (GenBank): CP001983.1 Gene position: 743244-745394 | *nasC* (small subunit) 99.2% (present) | |
| | | *Bacillus subtilis* subsp. *subtilis* 168 Accession number (KEGG2020): BSU03320 | | *nasB* (large subunit) 99.3% (present) |
| | | *Bacillus subtilis* subsp. *subtilis* 168: Accession number (KEGG2020): BSU03310 | | *nasC* (small subunit) 98.9% (present) |

\* Similarity of the sequence of the corresponding gene with sequences of the indicated strains.

**Nitrate reduction to ammonium**

Both strains CBLUT9 *(Bacillus subtilis)* and RPVPMO04 *(Bacillus megaterium)* were tested for the production of ammonium from nitrate. Both were found to produce ammonium when cultured in a medium with nitrate as only nitrogen source.

A bacterium harboring the assimilatory metabolic pathway of reduction of nitrate to ammonium (ANRA) can be detected by the presence of the ammonium ion, after incubation in a minimum growth medium containing nitrate as the sole nitrogen source. The ammonium ion can be detected even if it is an intermediate species, i.e.: ammonium ion is subsequently assimilated by the bacteria to be transformed in bacterial macromolecules (R-NH$_2$). The composition of the minimum growth medium was 0.6 g/l KH$_2$PO$_4$, 0.4 g/l MgSC$>_4$7H$_2$0, 4 g/l glucose, 8 g/l de mannitol, 8 g/l sodium pyruvate, 1 ml/l vitamins solution and 1 ml/l de trace elements (Bergersen et al., 1961. Aust J Biol Sci 14: 349-360), plus 1 g/l NO$_3$K as the only source of nitrogen. The test was carried out in duplicate, in test tubes with 5 ml of the aforementioned growth medium, incubating the bacteria at 28 °C for 72 h. The presence of ammonium was detected using a colorimetric commercial test kit. (VACUettes KIT Ammonia, CHEMetrics).

**Table 3.** Production of ammonium ion by both strains incubated in minimum growth medium (Bergersen et al. 1961. Aust J Biol Sci 14: 349-360), supplemented with 1 g/l $NO_3K$ as the only source of nitrogen. Semi-quantitative determination using commercial test kit. VACUettes KIT Ammonia, CHEMetrics.

| Strain | Identification | Ammonium ion (ppm in mg/l) |
|---|---|---|
| RPVPM004 | *Bacillus megaterium* | 45 |
| CBLUT-9 | *Bacillus subtilis* | 15 |

**Leaching prevention**

Both strains CBLUT9 *(Bacillus subtilis)* and RPVPMO04 *(Bacillus megaterium)* were effective in preventing leaching in column tests, in which the nitrogen leached from an agricultural soil was determined after successive irrigations over 60 days. The test was carried out by incorporating a mineral fertiliser (calcium ammonium nitrate 27 % nitrogen), coated with each one of the strains, into the upper soil layer of the leaching columns. A control without microorganisms was included. It was observed that the fertilisers coated with the strains reduced in both cases the leaching of nitrogen between 20 and 30 %.

Also, it was observed that the mineral nitrogen levels in the soil were higher when the strains were added to the soil, in comparison with the untreated control, as can be seen in fig. 1.

In this experiment 270 mg of N/kg of soil and $3 \times 10^2$ CFU/g of soil were applied.

The inventors also tested the fertiliser ammonium nitrate coated with both strains CBLUT9 *(Bacillus subtilis)* and RPVPMO04 *(Bacillus megaterium)* and checked if they were capable of reducing nitrates to ammonia in pots without plants subject to a leaching process. These tests allowed to analyse, in real soil conditions and without a plant, the effect on the leaching of the different forms of nitrogen, of the coating of the calcium ammonium nitrate fertiliser (27 % nitrogen content) with each strain that reduce nitrates to ammonium.

The treatment consisted in a fertiliser coated with each one of the strains CBLUT9 *(Bacillus subtilis)* and RPVPMO04 *(Bacillus megaterium)* at a dose of 1% (volume of culture broth/weight of fertiliser). The concentration of the culture broth was 6- 10ˢ CFU per ml in both cases. In both cases, the composition comprising the microorganism also comprised 1% w/w carob gum.

The nitrate leached after 5 and 20 days (mg/l in leachate) was tested for pots with each strain and with the fertiliser only as control and the following results were obtained, showing that both strains reduced nitrate leaching:

| Treatment | Wash at 5 days | Wash at 20 days |
|---|---|---|
| Control | 4100 | 3800 |
| CBLUT9 | 1200 | 1450 |
| RPVPMO04 | 1480 | 1650 |

In this experiment 225 mg of N/kg of soil and $6 \times 10^3$ CFU/g of soil were applied.

The inventors also tested the fertiliser ammonium sulphonitrate (ASN) coated with each one of the strains capable of reducing nitrates to ammonia in columns, subject to a leaching process. The negative control was soil, and the positive control was uncoated ASN. Both strains markedly reduced the amount of leached nitrate from the columns in two tests performed on different dates:

| Test | Treatment | Leached $NO_3^-$ (mg) |
|---|---|---|
| 1 | C- (soil) | 1172 |
| 1 | C+ (ASN) | 2631 |
| 1 | CBLUT9 | 1667 |
| 1 | RPVPMO04 | 1907 |
| 2 | C- (soil) | 1202 |
| 2 | C+ (ASN) | 3271 |
| 2 | CBLUT9 | 1771 |
| 2 | RPVPMO04 | 2175 |

In this experiment 148 mg of N/kg of soil and 1,71 $\times 10^2$ CFU/g of soil were applied.

**Biostimulant effect and increased fertilization efficiency**

Microcosmos tests with plant were performed in the following conditions:
Agricultural soil. pH: neutral. Fertiliser: NPK 22-10-6. Crop: corn. Duration: 5 weeks.

The results in the table below demonstrate the environmental effect by the lower $NO_3^-/NH_4^+$ ratio in the soil and the biostimulant effect, by the biomass results. Also, a greater efficiency of fertilization is observed. The two strains, CBLUT9 and RPVPMO04, were tested and also a control only with NPK fertiliser was included (NPK without microorganism), as well as a control were nothing was added to the plants (control 0).

| | CBLUT9 | RPVPMO04 | NPK without microorganism | Control 0 |
|---|---|---|---|---|
| Fresh weight (g/plant) | 25.2 (+11%) | 28.2 (+24%) | 22.8 | 11.3 |
| Dry weight (g/plant) | 2.8 (+17%) | 3.1 (+29%) | 2.4 | 1.3 |
| Plant height (cm) | 59.8 (+20%) | 53.1 (+6%) | 50.0 | 42.9 |
| Extraction of N (mg/plant) | 47.3 | 53.4 | 48.9 | 15.7 |
| Physiological efficiency of nitrogen (PE = (Y – Yo)/ (U – Uo) ; Y (yield, g of dry biomass), U (nitrogen uptake mg) | 0.047 (+42%) | 0.048 (+45%) | 0.033 | |
| Internal nitrogen utilization efficiency IE (increased production/nitrogen extracted) | 0.032 (+45%) | 0.034 (+55%) | 0.022 | |
| $NO_3^-/NH_4^+$ at the end of the test | 13.8 (-22%) | 13.2 (-25%) | 17.7 | |

PE: Physiological efficiency of nitrogen

Y: Yield (g of biomass).

Yo: Yield in the control 0 treatment (g of biomass)

U: Amount of nutrient acquired (nitrogen uptake) by the plant biomass (mg/plant)

Uo: Amount of nutrient acquired (nitrogen uptake) by the plant biomass of the Control 0 treatment (0 nitrogen added) (mg/plant)

In parenthesis, the percent difference with respect to the NPK without microorganism. In this experiment 50 mg of N/kg of soil and 1 ,37 $\times 10^3$ CFU/g of soil were applied.

**Biostimulatory effect**

A first field trial was performed in the following conditions:
Corn cultivation in Valladolid, Spain in year 2016. Fertiliser: NPK 18-8-10.

| | Relative performance against NPK (%) | AE |
|---|---|---|
| Control 0 | 51 | |
| NPK | 100 | 32 |
| NPK+CBLUT9 | 105 | 35 |
| NPK+ RPVPMO04 | 107 | 37 |

$$\text{Relative performance against NPK} = 100*(Y-Y_{NPK})/\ Y_{NPK}$$

AE = Agronomic efficiency of supplied nutrients (kg yield increase per kg nutrient applied) = (Y-Yo) / F
Y = Yield (corn) (kg/ha)
Yo = Yield in the Control 0 treatment (kg/ha)
$Y_{NPK}$ = Yield in the NPK treatment (kg/ha)
F = Amount of nutrient made available to the crop (kg fertilizer N / ha)

The biostimulatory effect results in a bigger harvest.
In this experiment 220 kg of N/ha and 5,5 $\times 10^1$ CFU/g of soil were applied.
A second field trial was performed in the following conditions:
Corn cultivation in Valladolid, Spain in year 2016. Fertiliser: NPK 20-5-10.

| | Average $N\text{-}NO_3^-$ throughout the crop cycle | Average $N\text{-}NO_3^-/N\text{-}NH_4^+$ throughout the crop cycle |
|---|---|---|
| C (NPK) | 35.0 | 21.0 |
| NPK+ CBLUT9 | 32.1 | 15.3 |
| Variation (%) | -8.3 | -26.6 |
| NPK+ RPVPMO04 | 31.3 | 13.4 |
| Variation (%) | -10.6 | -36.2 |

An environmental effect is demonstrated, with a lower concentration of nitrate in the soil and a lower NO3⁻/NH⁺ ratio.
In this experiment 160 kg of N/ ha and 5,5 $\times 10^1$ CFU/g of soil were applied.

**Reduction in the emission of $N_2O$**

In a trial under controlled conditions, in pots with moistened soil and without plants, $N_2O$ emissions were measured for 14 days after fertilizer application.
Strains CBLUT9 and RPVPMO04 were able to reduce the emission of $N_2O$ from the fertiliser when applied to a soil. We have found that adding nitrogen fertiliser with these strains reduces $N_2O$ emissions in both fertilisers used.

| Fertiliser | Strain | N$_2$O Accumulated emission (kg N$_2$O-N/ha) | N$_2$O emission reduction |
|---|---|---|---|
| ASN | None | 109.4 | |
| ASN | CBLUT9 | 84.3 | -23% |
| ASN | RPVPMO04 | 103.25 | -6% |
| CAN | None | 161.0 | |
| CAN | CBLUT9 | 123.1 | -24% |
| CAN | RPVPMO04 | 127.8 | -21% |

ASN: Ammonium nitrosulphate (26% Nitrogen)

CAN: Calcium ammonium nitrate (27% Nitrogen)

In this experiment 700 mg of N/kg of soil and 7,77 $\times 10^2$ CFU/g of soil were applied.

The incubation support consisted of pots with a diameter of 16 cm and a height of 11.5 cm. In each pot, 2300 g of agricultural soil were added in order to reach a bulk density of the soil of 1 g cm-3. The treatments were repeated 5 times (experimental blocks) and the pots were placed randomly in each of the blocks. On day 1, water was added to each of the pots until reaching 60% of the water-filled pore space (WFPS). Next, the different selected fertiliser products were applied to the pots. On the same day 1, 2 hours after applying the fertiliser products, a first measurement of N$_2$O was made. Over the next 14 days, a total of 10 N$_2$O measurements were made on different days. Sampling consisted of inserting a PVC chamber in each pot of 11.5 cm in diameter and 20 cm long (6 cm was inserted). Each chamber had an opening at the top that was closed during the sampling period with a hermetic PVC lid. These lids had a sampling hole covered with a rubber septum that allowed the insertion of a 6 ml syringe to sample the air inside the chamber. Once the chamber was closed, the air inside was sampled at the following two moments: when the lid was closed (Ti) and after 20 minutes (Tf). The air collected at each of the moments was stored in vacuum vials of the exetainer type. The concentration of N$_2$O stored in each of the vials was analysed using an Agilent 7890B gas chromatograph equipped with an electron capture detector (ECD) that works at a temperature of 280 °C and an HP-Plot Q column that uses He as carrier gas. The two N$_2$O concentration values inside each chamber, obtained for each sampling day, allowed the calculation of the daily emission flux from the soil to the atmosphere in each chamber. This flow value was expressed as mg N$_2$O-N m-2 day-1. Likewise, the integration of each of the daily values allowed calculating the accumulated value of N$_2$O emitted for each chamber during the course of the 14 days of the experiment.

## Claims

1. A fertiliser comprising a composition comprising at least one aerobic rhizospheric microorganism, wherein said microorganism comprises in its genome at least one nitrate reductase gene and at least one nitrite reductase gene and wherein said microorganism reduces nitrate to ammonium when cultured in a minimum growth medium with nitrate as only source of nitrogen, and wherein the fertiliser is selected from the group consisting of nitrogenated fertilisers, phosphated fertilisers, potassium fertilisers, NP compound fertilisers, PK compound fertilisers, NK compound fertilisers or NPK compound fertilisers, limestone amendments, magnesium amendments, sulphur amendments, calcium and sulphur amendments, moisture retainer amendments, silica amendments, and organic amendments.

2. The fertiliser according to the preceding claim, wherein the composition comprises from $10^3$ to $5 \cdot 10^{12}$ CFU per gram of composition, preferably from $10^5$ to $10^{11}$ CFU per gram of composition, more preferably from $10^6$ to $10^{10}$ CFU per gram of composition.

3. The fertiliser according to any one of the preceding claims, wherein said microorganism does not comprise in its genome the gene nrfA, and/or wherein said microorganism cannot produce N$_2$ from nitrates, and/or wherein said microorganism comprises at least one of genes nasB or nasC and at least one of genes nasD or nasE.

4. The fertiliser according to any one of the preceding claims, wherein said microorganism is gram +.

5. The fertiliser according to any one of the preceding claims, wherein said microorganism is a species of the *Bacillus* genus, preferably said microorganism is of the species *Bacillus subtilis* or *Bacillus megaterium.*

6. The fertiliser according to any one of the preceding claims, wherein said microorganism is strain CECT 30572, strain CECT 30573 or a combination of both.

7. The fertiliser according to any one of the preceding claims, wherein the fertiliser comprises from $10^2$ to $10^{10}$ CFU per gram of fertiliser, preferably from $10^3$ to $10^9$ CFU per gram of fertiliser, more preferably from $10^4$ to $10^8$ CFU per gram of fertiliser.

8. The fertiliser according to any one of the preceding claims, wherein the fertiliser is selected from the group consisting of phosphated fertilisers, potassium fertilisers, NP compound fertilisers, PK compound fertilisers, NK compound fertilisers or NPK compound fertilisers.

9. The fertiliser according to any one of the preceding claims, wherein the fertiliser is solid or liquid, mineral, organo-mineral or organic.

10. The fertiliser according to any one of the preceding claims, wherein the microorganism is protected by a microorganism-protective-compound, such as trehalose, carob gum or xanthan gum.

11. Use of a composition comprising at least one aerobic rhizospheric microorganism, wherein said microorganism comprises in its genome at least one nitrate reductase gene and at least one nitrite reductase gene and wherein said microorganism reduces nitrate to ammonium when cultured in a minimum growth medium with nitrate as only source of nitrogen, or of the fertiliser according to any one of claims 1 to 10, for preventing nitrogen leaching and/or for improving general crop performance or crop yield.

12. Use of the composition or fertiliser according to the preceding claim, wherein said composition or fertiliser is directly applied to the soil as is or in combination with an organic or inorganic carrier, or wherein said composition is applied to the soil in the irrigation water or in a compost or in a soil improver, or wherein said composition is applied to a seed before seeding.

13. Use of a composition comprising at least one aerobic rhizospheric microorganism, wherein said microorganism comprises in its genome at least one nitrate reductase gene and at least one nitrite reductase gene and wherein said microorganism reduces nitrate to ammonium when cultured in a minimum growth medium with nitrate as only source of nitrogen, or of the fertiliser according to any one of claims 1 to 10, for preventing the emission of $N_2O$.

14. A composition comprising at least one aerobic rhizospheric microorganism, wherein said microorganism comprises in its genome at least one nitrate reductase gene and at least one nitrite reductase gene and wherein said microorganism reduces nitrate to ammonium when cultured in a minimum growth medium with nitrate as only source of nitrogen, wherein said microorganism is strain CECT 30572, strain CECT 30573 or a combination of both.

15. Use of the composition according to claim 14 for preventing nitrogen leaching, for improving general crop performance or crop yield, or for preventing the emission of $N_2O$.

**Figure 1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 22758448 A

- EP 3085679 A

**Non-patent literature cited in the description**

- **BERGERSEN et al.** *Aust J Biol Sci*, 1961, vol. 14, 349-360